# EUROPÄISCHE PATENTANMELDUNG

(11) **EP 4 464 785 A1**
(43) Veröffentlichungstag der Anmeldung: **20.11.2024**
(21) Anmeldenummer: 23173450.0
(22) Anmeldetag: 15.05.2023
(51) Int. Cl.: C12P 19/02, C12N 9/04, C12N 9/02, A23L 27/30

(54) **VERFAHREN ZUR HERSTELLUNG VON D-TALITOL, D-TAGATOSE UND D-PSICOSE**

(71) Anmelder: Annikki GmbH, 8074 Raaba-Grambach (AT)
(72) Erfinder: Staunig, Nicole, 8074 Raaba-Grambach (AT); Dupont, Maria, 8074 Raaba-Grambach (AT); Trobe, Melanie, 8074 Raaba-Grambach (AT)
(74) Vertreter: Schwarz & Partner Patentanwälte GmbH

(57) **Zusammenfassung**

Die Erfindung besteht in einem Verfahren zur Herstellung einer wässerigen Lösung enthaltend D-Talitol, indem aus D-Fructose, welche in einer wässerigen Lösung gelöst vorliegt, durch Behandeln mit einer Epimerase *in vitro* D-Psicose gebildet wird, wonach die D-Psicose durch Behandeln mit einer NAD(P)H-abhängigen Oxidoreduktase *in vitro* zu D-Talitol reduziert wird. Das in der wässerigen Lösung enthaltene D-Talitol kann auf einfache Weise durch Behandeln mit einer Oxidoreduktase zu D-Tagatose oder D-Psicose oxidiert werden.

## Beschreibung

Die vorliegende Erfindung betrifft die Herstellung von D-Talitol. Die Erfindung betrifft ferner die Herstellung einer wässerigen Lösung enthaltend D-Tagatose oder D-Psicose.

### Hintergrund der Erfindung

Der sechswertige Zuckeralkohol D-Talitol (auch bekannt als D-Altritol) gehört aufgrund seines geringen natürlichen Vorkommens (z.B. in Braunalgen wie *Himanthalia elongata* (Reed et al., 1995)) zu den sogenannten seltenen Zuckern. In der sogenannten Izumoring-Strategie nimmt D-Talitol eine wichtige Rolle ein, da dessen Oxidation zu den Ketohexosen D-Tagatose und D-Psicose sowie zu den Aldohexosen D-Altrose und D-Talose führt, die ebenfalls zu den seltenen Zuckern gezählt werden (Izumori, 2006).

D-Tagatose findet sich unter anderem im Gummi des Tropenbaums *Sterculia setigera* (Hirst et al., 1949), in der Flechte *Roccella* (Lindberg, 1955) und in stark erhitzter Milch (Adachi, 1958). D-Psicose konnte in den Blättern von Rosmarinweiden (*Itea* sp.) nachgewiesen werden (Hough & Stacey, 1963), findet sich aber auch in verarbeiteten Lebensmitteln wie Konfekt und Gewürzsaucen, wo sie unter Einwirkung von Hitze aus D-Fructose, ihrem C3-Epimer, entsteht (Oshima et al., 2006).

Sowohl D-Tagatose als auch D-Psicose zeichnen sich durch ihren süßen Geschmack bei einem deutlichen geringeren Energiegehalt (bezogen auf Saccharose) aus (Jiang et al., 2020; Oh, 2007; Roy et al., 2018), was beide Zucker als Süßungsmittel für die Lebensmittelindustrie besonders interessant macht.

D-Tagatose und D-Psicose sind als *Generally Recognized as Safe* (GRAS)-Süßungsmittel von der USamerikanischen Lebensmittelaufsichtsbehörde Food and Drug Administration (FDA) zugelassen, in der Europäischen Union hingegen findet sich nur D-Tagatose auf der sogenannten *Novel* Foods-Liste und darf damit in der EU als Süßungsmittel verwendet werden (Levin, 2002; Ahmed et al., 2022).

D-Tagatose kann auch als Fungizid eingesetzt werden - so inhibiert sie den D-Mannose-Metabolismus von Pilzen und Oomyceten und wirkt damit gegen den Falschen Mehltau (Mochizuki et al., 2020). US 10638752 B2 sowie Chaled et al. (2021) beschreiben den Einsatz von D-Tagatose gegen diverse Erreger von Pflanzenkrankheiten.

D-Psicose beeinflusst den Lipid-Stoffwechsel sowie den Kohlenhydrat-Stoffwechsel (z.B. antidiabetisch) positiv und wirkt entzündungshemmend sowie antioxidativ (Zhang et al., 2016; Jiang et al., 2020; Chen et al., 2022).

Zur Herstellung von D-Tagatose existieren einige chemische oder biotechnologische Routen wie z.B. die baseninduzierte Isomerisierung von D-Galactose gewonnen aus Lactose (EP 0518874 B1; US 9150938 B2; US 8802843 B2), die enzymatische Isomerisierung von D-Galactose mittels L-Arabinose-Isomerase (Heath et al., 1958; Oh, 2007; Roy et al., 2018), die Herstellung ausgehend von Mono-, Di-, Oligo- oder Polysacchariden über phosphorylierte Intermediate mit der C4-Epimerisierung von D-Fructose-6-phosphat zu D-Tagatose-6-phosphat als Hauptschritt (EP 3322803 B1; US 10138506 B2; US 11034988 B2; Dai et al., 2020; Lee et al., 2017) oder die Oxidation des Zuckeralkohols Galactitol mit Galactitol-2-Dehydrogenase (EC 1.1.1.16) (Roy et al., 2018; Shaw, 1956).

D-Psicose wiederum kann durch Epimerisierung von D-Fructose mit einer Ketose-3-Epimerase gewonnen werden (Izumori et al., 1993). Ketose-3-Epimerasen lassen sich je nach Substratspezifität in drei Gruppen einteilen: 1) D-Tagatose-3-Epimerase (DTE), 2) D-Psicose-3-Epimerase (DPE) oder D-Allulose-3-Epimerase (DAE) und 3) L-Ribulose-3-Epimerase (LRE) (Zhang et al., 2016; Jiang et al., 2020).

Die Umsetzung von D-Fructose zu D-Psicose mittels Ketose-3-Epimerasen läuft jedoch nicht vollständig ab, vielmehr bildet sich ein Gleichgewichtsverhältnis zwischen beiden Epimeren aus. Je nach Reaktionsbedingungen (Temperatur zwischen 40 und 70 °C, pH-Wert zwischen 6 und 11) liegt dieses zwischen 80:20 und 62,5:37,5 (D-Fructose : D-Psicose). Viele der Epimerasen benötigen außerdem ein zweiwertiges Metallion wie Mn²⁺ oder Co²⁺ (toxisch) als Kofaktor (Zhang et al., 2016; Jiang et al., 2020).

Die bei der Epimerisierung von D-Fructose entstehenden D-Fructose/D-Psicose-Mischungen müssen entweder durch chromatographische Verfahren oder durch die "biologische Methode" (Fermentation der überschüssigen D-Fructose zu z.B. Ethanol) getrennt werden (Jiang et al., 2020). In US 2021/0189441 A1 wird die überschüssige D-Fructose durch einen probiotischen Mikroorganismus (*Lactobacillus* oder *Saccharomyces*) in L-Milchsäure umgewandelt. EP 3423460 B1 beschreibt ein Verfahren mit lonenaustausch und kontinuierlicher Chromatographie zur Aufreinigung einer D-Fructose/D-Psicose-Mischung und zur Gewinnung hochreiner D-Psicose. EP 3553069 A1 sowie Van Duc Long et al. (2009) legen eine Methode zur Trennung von D-Psicose und D-Fructose basierend auf der Simulated Moving Bed-Chromatographie offen.

Zur Herstellung von D-Psicose existieren noch einige weitere biotechnologische Routen wie z.B. die Herstellung ausgehend von Mono-, Di-, Oligo- oder Polysacchariden über phosphorylierte Intermediate mit der C3-Epimerisierung von D-Fructose-6-phosphat zu D-Psicose-6-phosphat als Hauptschritt (Li et al., 2021; US 11168342 B2; US 10907182 B2) oder die Aldol-Reaktion von Dihydroxyacetonphosphat und D-Glyceraldehyd mit anschließender Dephosphorylierung von D-Psicose-1-phosphat (Wang et al., 2020; Li et al., 2020).

Die unvorteilhafte Lage des Gleichgewichts der Epimerisierung von D-Fructose zu D-Psicose kann auch durch nachgeschaltete Reaktionen günstig beeinflusst werden. Eine Möglichkeit ist die gezielte Phosphorylierung von D-Psicose zu D-Psicose-1-phosphat mit L-Rhamnulose-Kinase und anschließender Dephosphorylierung mit einer sauren Phosphatase (Xiao et al., 2019). Der Nachteil an diesem Verfahren ist der stöchiometrische Verbrauch des teuren Phosphat-Donors Adenosintriphosphat (ATP).

Eine Alternative dazu ist die Kombination der Epimerase mit einer Ribitol-Dehydrogenase (RDH) und Formiat-Dehydrogenase (FDH; oxidiert Formiat zu CO₂) als Regenerationsenzym für den Kofaktor Nicotinamidadenindinukleotid (NADH), mit der D-Fructose über D-Psicose zu Allitol umgewandelt wird (Takeshita et al., 2000). Durch einen anschließenden Oxidationsschritt kann Allitol wieder zu D-Psicose umgesetzt werden.

Durch Hydrierung von D-Psicose mit Raney-Nickel oder Metallen der Platingruppe als Katalysator erhält man eine Mischung der beiden Epimere Allitol und D-Talitol, die chromatographisch getrennt werden kann (US 2004/0143024 A1).

Die Verwendung von Mikroorganismen erlaubt die stereoselektive Reduktion von Ketosen zu Zuckeralkoholen (bevorzugte Bildung eines der beiden Epimere). D-Psicose kann beispielsweise mit dem halotoleranten Hefe-Stamm *Candida famata* R28 (Sasahara et al., 1998) oder mit weiteren Pilzen (JP 2020039301 A; JP 2006166718 A) zu D-Talitol reduziert werden. D-Talitol kann aber auch durch Reduktion von D-Tagatose mit dem Pilz *Aureobasidium pullulans* (Stamm 113B) (Muniruzzaman et al., 1994) hergestellt werden.

Die Oxidation von D-Talitol zu D-Psicose kann beispielsweise mit dem Bakterium *Alcaligenes* sp. 701B durchgeführt werden (Izumori et al., 1990) und für die Oxidation zu D-Tagatose können beispielsweise Pilze aus der Familie *Mucoraceae* wie z.B. *Rhizopus oryzae* MYA-2483 verwendet werden, die D-Psicose auch direkt zu D-Tagatose umwandeln können (Yoshihara et al., 2006). Auch die D-Altritol-5-Dehydrogenase (EC 1.1.1.407), die Teil des D-Altritol-Katabolismus in *Agrobacterium tumefaciens* C58 ist, katalysiert die Oxidation von D-Talitol zu D-Tagatose (Wichelecki et al., 2015).

D-Tagatose kann durch C4-Epimerisierung direkt aus D-Fructose hergestellt werden. Durch gezielte Optimierungen des Enzyms Tagaturonat-3-Epimerase aus *Thermotoga petrophila* konnte eine Tagatose-4-Epimerase (T4E) entwickelt werden, welche 213 g/l D-Tagatose aus 700 g/l D-Fructose in 2 h bei 80 °C in Anwesenheit von 1,5 mM Ni²⁺ produzierte (Shin et al., 2020). Auch weitere aus thermophilen Mikroorganismen stammende Epimerasen sind bekannt (z.B. EP 3006568 B1; EP 3211078 B1; US 10196626 B2; US 11180785 B2; US 11408017 B2; EP 3677675 A1), diese benötigen aber auch erhöhte Reaktionstemperaturen (≥ 50 °C) und Zusatz von Zn-, Ni- oder Mn-lonen für eine optimale Aktivität.

In einer aktuellen Studie wurden Tagatose-4-Epimerasen aus *Thermotoga neapolitana* sowie aus *Kosmotoga olearia* getestet. Dazu wurden diese in *Corynebacterium glutamicum* exprimiert und in Form von Ganzzell-Biokatalysatoren eingesetzt. Auf diese Weise konnten Umsätze von 13,8% D-Tagatose (T4E aus *T. neapolitana*) bzw. 14,4% D-Tagatose (T4E aus K. *olearia;* 21,7% nach Codon-Optimierung) erreicht werden (30 g/l D-Fructose als Substrat, 60 °C für 2,5 h), jedoch müssen ebenfalls Schwermetallsalze (1 mM NiSO₄ für *T. neapolitana* und 3 mM CoSO₄ für *K. olearia*) zugesetzt werden (Jeon et al., 2023).

Da es sich bei D-Tagatose um ein Süßungsmittel handelt, sind Schwermetallionen im fertigen Produkt unerwünscht. Die höheren Reaktionstemperaturen sowie der nicht vollständige Umsatz sind weitere Nachteile der C4-Epimerisierung.

Wang et al. (2023) beschreiben ein System bestehend aus zwei E. coli-Ganzzell-Biokatalysatoren zur Umwandlung von D-Fructose (90 g/l) zu D-Psicose mit einem Umsatz von 90%. Im ersten Schritt (Umsetzung von D-Fructose zu Allitol) wurden *E. coli*-Zellen, welche eine DPE aus *Clostridiales,* eine RDH aus *Providencia alcalifaciens,* eine FDH aus *Starkeya* sowie eine weitere DPE aus *Rhizobium straminoryzae* beinhalteten, verwendet. Auf diese Weise wurde D-Fructose (500 mM = 90 g/l) innerhalb von 12 h bei 37 °C und pH 6 unter Einsatz von 1000 mM Natrium-Formiat (zwei Äquivalente bezogen auf D-Fructose) zu 452 mM Allitol umwandelt (Umsatz 90,4%). Als Nebenprodukt entstand ca. 30 mM D-Sorbitol (das Reduktionsprodukt der D-Fructose). Die Zellen wurden durch Zentrifugation abgetrennt und ausgetretene Proteine im Allitol-haltigen Überstand wurden durch Hitzeeinwirkung deaktiviert. Danach wurden *E. coli*-Zellen, welche eine RDH aus *Rubrivivax* sp. und eine NADH-Oxidase aus *Streptococcus pyogenes* beinhalteten, zur Allitol-Lösung zugesetzt. Allitol (452 mM) wurde innerhalb von 24 h bei pH 7 zu D-Psicose (450 mM) und das Nebenprodukt D-Sorbitol wurde wieder zu D-Fructose oxidiert. Der Gesamtumsatz des Verfahrens betrug also 90%.

Hier setzt nun die Aufgabe der vorliegenden Erfindung an. Die Erfindung möchte basierend auf den zuvor beschriebenen biotechnologischen Routen effiziente Verfahren zur Herstellung von wässrigen Lösungen von D-Tagatose und/oder D-Psicose mit hohem Umsatz zur Verfügung stellen, welche über die Zwischenstufe D-Talitol ablaufen und insbesondere im Eintopf-Verfahren durchgeführt werden können.

### Detaillierte Beschreibung der Erfindung

Die Aufgabe zur Herstellung von D-Talitol wird erfindungsgemäß gelöst, indem zunächst aus D-Fructose, welche in einer wässerigen Lösung gelöst vorliegt, durch Behandeln mit einer Epimerase *in vitro* D-Psicose gebildet wird, wonach die D-Psicose durch Behandeln mit einer NAD(P)H-abhängigen Oxidoreduktase *in vitro* zu D-Talitol reduziert und die Epimerase und die Oxidoreduktase abgetrennt werden. Aus der erhaltenen Lösung kann D-Talitol durch Kristallisation gewonnen werden.

Die Aufgabe zur Herstellung einer wässerigen Lösung von D-Tagatose oder D-Psicose wird gelöst, indem die nach den oben beschriebenen Verfahren hergestellte, wässerige, D-Talitol enthaltende Lösung mit einer NAD(P)⁺-abhängigen Oxidoreduktase behandelt wird, um D-Talitol zu D-Tagatose bzw. D-Talitol zu D-Psicose zu oxidieren, wonach die Oxidoreduktase abgetrennt wird.

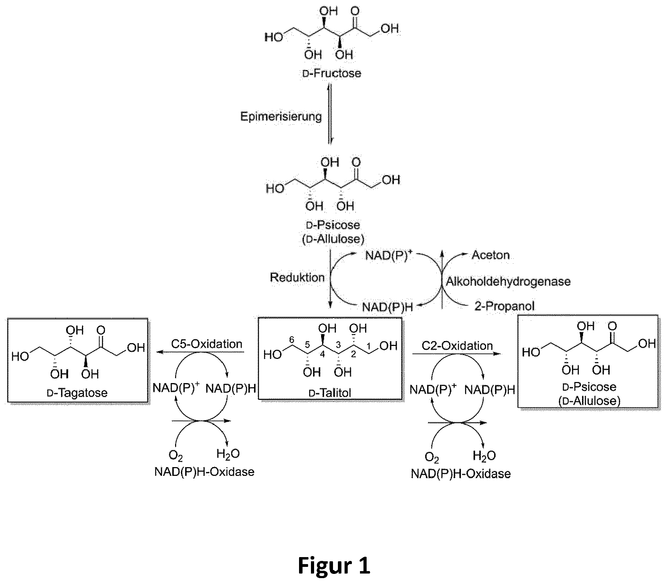

In der beiliegenden Figur ist das Reaktionsschema der erfindungsgemäßen Verfahren wiedergegeben.

Im Fall von D-Psicose werden vor der Ausführung des letzten Schritts (Oxidation) die Enzyme (Epimerase und Reduktase/Dehydrogenase) des ersten Schritts (D-Fructose → D-Talitol) durch Hitzeeinwirkung deaktiviert oder durch Ultrafiltration entfernt, da ohne die entsprechende Behandlung ein Großteil der durch die Oxidation entstandenen D-Psicose von der Epimerase wieder zu D-Fructose umgewandelt werden würde.

Eine bevorzugte Ausführungsform des erfindungsgemäßen Verfahrens zur Herstellung einer wässerigen Lösung enthaltend D-Talitol besteht darin, dass der durch die Reduktion entstandene oxidierte Kofaktor NAD(P)⁺ mittels einer Alkoholdehydrogenase und einem sekundären Alkohol unter Bildung eines Ketons reduziert wird, wie z.B. in EP 2812439 B1 beschrieben.

Es hat sich ferner gezeigt, dass der sekundäre Alkohol 2-Propanol (Isopropanol) besonders geeignet ist, weil das bei der Regeneration des Kofaktors gebildete Aceton auf einfache Weise aus dem Reaktionsgemisch entfernt werden kann.

Eine bevorzugte Ausführungsform dieses erfindungsgemäßen Verfahrens besteht darin, dass der durch die Oxidation entstandene, reduzierte Kofaktor NAD(P)H mittels einer Oxidase und Sauerstoff zu NAD(P)⁺ oxidiert wird.

Für die Regeneration der bei den Redoxreaktionen benötigten Kofaktoren wird im Falle der Reduktion der D-Psicose zu D-Talitol eine NAD(P)-abhängige Alkoholdehydrogenase und im Falle der Oxidation des Zuckeralkohols zu D-Tagatose oder D-Psicose eine H₂O-bildende NAD(P)H-Oxidase verwendet.

Eine weitere bevorzugte Variante der erfindungsgemäßen Verfahren ist dadurch gekennzeichnet, dass sie als Eintopf-Reaktion ohne Isolierung von Zwischenprodukten durchgeführt werden.

Die Abtrennung der Enzyme kann z.B. durch Zentrifugieren und/oder Ultrafiltrieren bewerkstelligt werden.

Die besonders bevorzugte Konzentration von D-Fructose beträgt 50 - 250 g/l.

Der besonders bevorzugte Temperaturbereich für den ersten Schritt (Epimerisierung und Reduktion) liegt zwischen 25 und 40 °C, für den zweiten Schritt (Oxidation) zwischen 20 und 35 °C.

Der besonders bevorzugte pH-Bereich beider Schritte liegt zwischen 7,0 und 8,5.

Bei einer weiteren, bevorzugten Variante des erfindungsgemäßen Verfahrens liegen die Enzyme in einer Suspension, im Homogenat und/oder im Lysat der entsprechenden, sie bildenden Zellen vor, wobei Lysate besonders bevorzugt sind.

Suspension bedeutet in diesem Kontext eine Suspension von *resting cells.* Diese werden nach der Kultivierung geerntet (vom Nährmedium abgetrennt) und in einem geeigneten Puffersystem suspendiert. Im Gegensatz zu fermentativen Verfahren, bei denen auch mit ganzen Zellen gearbeitet wird, können die *resting cells* aufgrund der Entfernung von Kohlenstoff-Quellen und Nährstoffen nicht mehr wachsen, sondern dienen nur der Umsetzung von Substraten (Lin & Tao, 2017). Homogenat steht in diesem Kontext für eine physikalisch und/oder chemisch behandelte Suspension (z.B. mittels Druckes, Lysozym oder Ultraschall behandelt), wobei die Zellbestandteile aus den Zellen freigesetzt werden. Ein Lysat wird erhalten, wenn die unlöslichen Zellbestandteile des Homogenats beispielsweise durch Filtration oder Zentrifugation entfernt werden (siehe *Produktion der Enzyme & Herstellung der Lysate* für Details).

In einer weiteren Variante können die Enzyme auch auf einem festen Trägermaterial immobilisiert sein.

In einer besonders bevorzugten Ausführung des Verfahrens werden für die Konversion des Startmaterials nur Enzyme aus den Enzymgruppen Epimerasen und Oxidoreduktasen verwendet, wobei eines oder mehrere dieser Enzyme aus jeder dieser Gruppen ausgewählt werden.

Die im Verfahren verwendete Epimerase kann aus einer der Gruppen EC 5.1.3.30 (D-Psicose-3-Epimerase) oder EC 5.1.3.31 (D-Tagatose-3-Epimerase/L-Ribulose-3-Epimerase) stammen, wobei die erstgenannte besonders bevorzugt ist.

Die NAD(P)H-abhängige Oxidoreduktase zur Reduktion von D-Psicose zu D-Talitol umfasst vorzugsweise eine Aminosäuresequenz, welche ausgewählt ist aus der Gruppe bestehend aus:
i) einer Aminosäuresequenz, die eine Identität zu SEQ ID Nr. 2 von mindestens 80% aufweist,
ii) einer Aminosäuresequenz, die durch eine Nukleinsäure kodiert wird, die eine Identität zu SEQ ID Nr. 1 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet.

SEQ ID Nr. 1: 
ATGAACGATCCCGTTCTGGAAGCCAGACCGATTTTGGAGAGATTTCGCCTCGACGGCCGCGTGGCGC
TGGTGACCGGCGGTGGGCAGGGCATCGGCCGCGCCTTCGCCCATGCGCTGTGCGAGGCAGGCGCCG
CAGTGGCCGTCGTCGATCTGCGCCTGGACCTGGCCGAGGAAGTGGCGCATGAGCTGGTAAACAAGC
AGATCGACGCCATCGCAATTCAGGCCGATGTGACGAAGCCCGATCAGGTGCAGACGATGGTTGATGC
CATTCTGTCCAGGTGGGGGACGTTGACGATCGGTGTCAACAACGCCGGCATCGGCTTGTGGGCGGAT
GCCGAAAGCATGGCATACACAGACTGGCTGCGCGTGATCGATATTGATCTGAACTCGGTTTTCCTCTG
CGCCCAGGCTGAAGCGAGGGTCATGCTGCCGGCCGGCTACGGCAAGATCATCAACACGGCGTCGAT
GTCCGGCCATATCAGCAACACGCCGCAAAATCAGGCTGCTTACAACACGGCCAAGGCTGGCGTGATC
CATCTGACGCGCAGCCTTGCCGCGGAATGGGCCAAGCGGGGAGTGCGCGTCAACAGCATCAGCCCC
GGCTATACGCGCACCAAATTGGTTGACGACCTGCTCGCCACGCCGATCGGACAGACGATGTTGCCCA
CCTGGATGGGCATGACGCCCATGGGACGAATGGCCGAGGTCACCGATCTGCAAGGCGCCGTCGTCT
ACCTGGCGTCGTCGGCGTCCGACTATATGACCGGCCATGACATGGTGATTGACGGCGGCTATTGTTG
CTGGTAA
SEQ ID Nr. 2:

MNDPVLEARPILERFRLDGRVALVTGGGQGIGRAFAHALCEAGAAVAVVDLRLDLAEEVAHELVNKQIDA

IAIQADVTKPDQVQTMVDAILSRWGTLTIGVNNAGIGLWADAESMAYTDWLRVIDIDLNSVFLCAQAEA

RVMLPAGYGKIINTASMSGHISNTPQNQAAYNTAKAGVIHLTRSLAAEWAKRGVRVNSISPGYTRTKLVD

DLLATPIGQTMLPTWMGMTPMGRMAEVTDLQGAVVYLASSASDYMTGHDMVIDGGYCCW

Es hat sich überraschenderweise gezeigt, dass eine Oxidoreduktase mit einer der oben genannten Aminosäuresequenz nicht nur in der Lage ist, D-Psicose zu D-Talitol zu reduzieren, wobei die Reaktion vorzugsweise in Anwesenheit von NAD(P)H stattfinden kann, sondern auch D-Talitol zu D-Psicose zu oxidieren, wobei diese Reaktion vorzugsweise in Anwesenheit von NAD(P)⁺ stattfinden kann.

Die Oxidoreduktase zur Bildung von D-Talitol aus D-Psicose bzw. von D-Psicose aus D-Talitol umfasst vorzugsweise eine Aminosäuresequenz, die eine Identität zu SEQ ID Nr. 2 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die erfindungsgemäße Oxidoreduktase zur Bildung von D-Talitol aus D-Psicose die Aminosäuresequenz SEQ ID Nr. 2 oder besteht aus dieser.

Alternativ, umfasst die Oxidoreduktase zur Bildung von D-Talitol aus D-Psicose bzw. von D-Psicose aus D-Talitol vorzugsweise eine Aminosäuresequenz, die durch eine Nukleinsäure kodiert wird, die eine Identität zu SEQ ID Nr. 1 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die erfindungsgemäße Oxidoreduktase zur Bildung von D-Talitol aus D-Psicose bzw. von D-Psicose aus D-Talitol kodiert, die Nukleinsäuresequenz SEQ ID Nr. 1 oder besteht aus dieser.

Der Begriff "Identität", wie hier verwendet, bezieht sich auf den Prozentsatz der identischen Nukleotid- bzw. Aminosäureübereinstimmungen zwischen mindestens zwei, unter Verwendung eines standardisierten Algorithmus miteinander ausgerichteten Nukleotid- bzw. Aminosäuresequenzen ("Alignment"). Ein solcher Algorithmus kann, in einer standardisierten und reproduzierbaren Weise, Lücken ("Gap") in die verglichenen Sequenzen einfügen, um das Alignment zwischen zwei Sequenzen zu optimieren, und somit einen aussagekräftigeren Vergleich der beiden Sequenzen erreichen.

Die prozentuale Identität zwischen Sequenzen kann unter Verwendung von ein oder mehreren Computeralgorithmen oder im Stand der Technik bekannten oder hierin beschriebenen Programmen bestimmt werden. Erfindungsgemäß wird zur Bestimmung der Identität das durch National Center for Biotechnology Information (NCBI) bereitgestellte Basic Local Alignment Search Tool (BLAST) (Altschul et al., 1990) verwendet. Die BLAST-Software-Reihe enthält verschiedene Programme, einschließlich eines als "BLAST 2 Sequences" genannten Werkzeuges, das für den direkten paarweisen Vergleich von zwei Nukleotid- bzw. Aminosäuresequenzen verwendet wird. "BLAST 2 Sequences" kann auch über die NCBI World Wide Web-Seite im Internet interaktiv abgerufen und verwendet werden. Das blastn-Programm (für Nukleotidsequenzen) verwendet als Vorgaben eine Wortlänge (W) von 11, eine Erwartung (E) von 10, M = 5, N = -4 und einen Vergleich beider Stränge. Für Aminosäuresequenzen verwendet das blastp-Programm als Vorgaben eine Wortlänge von 3 und eine Erwartung (E) von 10 und die BLOSUM62-Scoring-Matrix (Henikoff & Henikoff, 1989), Alignments (B) von 50, Erwartung (E) von 10, M = 5, N = -4.

Alternativ, umfasst die Oxidoreduktase zur Bildung von D-Talitol aus D-Psicose bzw. von D-Psicose aus D-Talitol vorzugsweise eine Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet. Wie hierin verwendet, beziehen sich die stringenten Bedingungen auf Bedingungen, unter denen sogenannte spezifische Hybride, jedoch keine unspezifischen Hybride gebildet werden. Beispielsweise umfassen die stringenten Bedingungen eine Hybridisierung in 6xSSC (Natriumchlorid/Natriumcitrat) bei 45°C und dann Waschen mit 0,2 bis 1xSSC, 0,1% SDS bei 50 bis 65°C; oder derartige Bedingungen können eine Hybridisierung in 1xSSC bei 65 bis 70°C und dann Waschen mit 0,3xSSC bei 65 bis 70°C umfassen. Die Hybridisierung kann durch herkömmlich bekannte Verfahren, wie etwa jene, die von J. Sambrook et al. in Molecular Cloning, A Laboratory Manual, 2nd Ed., Cold Spring Harbor Laboratory (1989), beschrieben sind, durchgeführt werden.

Ein weiterer Aspekt der vorliegenden Erfindung betrifft ein Verfahren zur Herstellung von D-Talitol oder D-Psicose umfassend den Schritt des Behandelns von D-Psicose bzw. D-Talitol mit einer Oxidoreduktase, die eine Aminosäuresequenz umfasst, welche ausgewählt ist aus der Gruppe bestehend aus:
i) einer Aminosäuresequenz, die eine Identität zu SEQ ID Nr. 2 von mindestens 80% aufweist,
ii) einer Aminosäuresequenz, die durch eine Nukleinsäure kodiert wird, die eine Identität zu SEQ ID Nr. 1 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet.

Ein noch weiterer Aspekt der vorliegenden Erfindung betrifft eine Oxidoreduktase zur Reduktion von D-Psicose zu D-Talitol und/oder zur Oxidation von D-Talitol zu D-Psicose umfassend eine Aminosäuresequenz, welche ausgewählt ist aus der Gruppe bestehend aus:
i) einer Aminosäuresequenz, die eine Identität zu SEQ ID Nr. 2 von mindestens 80% aufweist,
ii) einer Aminosäuresequenz, die durch eine Nukleinsäure kodiert wird, die eine Identität zu SEQ ID Nr. 1 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet.

Die NAD(P)⁺-abhängige Oxidoreduktase zur Oxidation von D-Talitol zu D-Tagatose umfasst vorzugsweise eine Aminosäuresequenz, welche ausgewählt ist aus der Gruppe bestehend aus:
i) einer Aminosäuresequenz, die eine Identität zu SEQ ID Nr. 4 von mindestens 80% aufweist,
ii) einer Aminosäuresequenz, die durch eine Nukleinsäure kodiert wird, die eine Identität zu SEQ ID Nr. 3 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 3 bindet.

SEQ ID Nr. 3: 
ATGGCTGCCAACGTCCCCAAGACCATGAAGGCTCTCAGATATGAGAAGCCTGAGACGTTCTCCATCGTCGACA
TTCCCGTTCCCACTCTGCGTGAGAACGATGTCTTGATCAAGGTCAAGGCTTGCGGTGTCTGTGGTACCGACCTG
CACATTCACGAGGGAGAATTCCTTGCCAAGTTCCCTCTCGTTCCTGGCCACGAGACTGTCGGTGTTGTTGCCGC
AGTTGGACCCAAGGTCAAGGGTTTCGAGATCGGTGACCGTGTTGTTGCCGACAACTCCGAGCTTTGCGGCCAA
TGCTTCTACTGCCGACGAGGAGAGGAGTTGCTCTGCGAGCACTTCGAAGCTCACGGTGTCACGATGAACGGC
GGTTTCGCTGAGTACTGCGCCTACCCTGCCGGCCGTGTCTTCAAGATCAAGAACCTCTCTGACGTGGACGCCAC
TCTGCTTGAGCCCGCGTCCTGCGCCGCTCACGGTCTGGACAAGATTGCCCCCAAGATGGGCTCGTCCGTCCTG
GTGTTCGGCGCCGGTCCCACCGGTCTGGTCCTTGCTCAGATGCTCCGTCTGAACGGAGGATGCCGCGTCGTCG
TCGCTGCGCCCGAGGGTCTGAAGATGGACCTGGCCCAGAAGCTCGGCGCTGGTGATGAATACGTTGCTCTTTC
TCGCACGAACCCTCAGGCTCAGTTTGACAAGCTGAAGGCCGACAACCCGTACGGCTTCGACATTGTCGTCGAG
GCTACCGGCAATGCCAAGATCCTGGAGGATGCCATCAACTATGTCCGCCGTGGAGGCAAACTGGTCGTGTACG
GTGTGTACGCGAACAAGGACCGCGTCTCGTGGCCCCCGAGCAAGATCTTCGGTGACGAAATCACCATTCTGGG
TAGCTTCTCCGAGACCTACAAGTTCCCCGCCGCCATCGACTACCTGGACTCCGGCAAGGTGAAGGTCCAGGGC
ATCGTGAACAAGACCTTCCGGCTGGAGCAGTGGGAGGAGTGTCTGGCGTCGTTGAAGAACAAGAGCGCCATC
AAGGCGGCGATCGTCTTTGACTAA
SEQ ID Nr. 4:

MAANVPKTMKALRYEKPETFSIVDIPVPTLRENDVLIKVKACGVCGTDLHIHEGEFLAKFPLVPGHETVGVVAAVGP
KVKGFEIGDRVVADNSELCGQCFYCRRGEELLCEHFEAHGVTMNGGFAEYCAYPAGRVFKIKNLSDVDATLLEPASC
AAHGLDKIAPKMGSSVLVFGAGPTGLVLAQMLRLNGGCRVVVAAPEGLKMDLAQKLGAGDEYVALSRTNPQAQF
DKLKADNPYGFDIVVEATGNAKILEDAINYVRRGGKLVVYGVYANKDRVSWPPSKIFGDEITILGSFSETYKFPAAIDY
LDSGKVKVQGIVNKTFRLEQWEECLASLKNKSAIKAAIVFD

Es hat sich überraschenderweise gezeigt, dass eine Oxidoreduktase mit einer der oben genannten Aminosäuresequenz in der Lage ist, D-Talitol zu D-Tagatose zu oxidieren, wobei die Reaktion vorzugsweise in Anwesenheit von NAD(P)⁺ stattfinden kann.

Die Oxidoreduktase zur Bildung von D-Tagatose aus D-Talitol umfasst vorzugsweise eine Aminosäuresequenz, die eine Identität zu SEQ ID Nr. 4 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die erfindungsgemäße Oxidoreduktase zur Bildung von D-Tagatose aus D-Talitol die Aminosäuresequenz SEQ ID Nr. 4 oder besteht aus dieser.

Alternativ umfasst die Oxidoreduktase zur Bildung von D-Tagatose aus D-Talitol vorzugsweise eine Aminosäuresequenz, die durch eine Nukleinsäure kodiert wird, die eine Identität zu SEQ ID Nr. 3 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die erfindungsgemäße Oxidoreduktase zur Bildung von D-Tagatose aus D-Talitol kodiert, die Nukleinsäuresequenz SEQ ID Nr. 3 oder besteht aus dieser.

Alternativ umfasst die Oxidoreduktase zur Bildung von D-Tagatose aus D-Talitol vorzugsweise eine Aminosäuresequenz, die durch eine Nukleinsäure kodiert wird, die unter stringenten Bedingungen (siehe obige Definition) an ein Nukleinsäuremolekül mit der Nukleinsäuresequenz SEQ ID Nr. 3 bindet.

Ein weiterer Aspekt der vorliegenden Erfindung betrifft ein Verfahren zur Herstellung von D-Tagatose umfassend den Schritt des Behandelns von D-Talitol mit einer Oxidoreduktase, die eine Aminosäuresequenz umfasst, welche ausgewählt ist aus der Gruppe bestehend aus:
i) einer Aminosäuresequenz, die eine Identität zu SEQ ID Nr. 4 von mindestens 80% aufweist,
ii) einer Aminosäuresequenz, die durch eine Nukleinsäure kodiert wird, die eine Identität zu SEQ ID Nr. 3 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 3 bindet.

Ein noch weiterer Aspekt der vorliegenden Erfindung betrifft eine Oxidoreduktase zur Oxidation von D-Talitol zu D-Tagatose umfassend eine Aminosäuresequenz, welche ausgewählt ist aus der Gruppe bestehend aus:
i) einer Aminosäuresequenz, die eine Identität zu SEQ ID Nr. 4 von mindestens 80% aufweist,
ii) einer Aminosäuresequenz, die durch eine Nukleinsäure kodiert wird, die eine Identität zu SEQ ID Nr. 3 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 3 bindet.

Die zur Kofaktor-Regeneration eingesetzte Alkoholdehydrogenase (ADH) kann aus einer der Gruppen EC 1.1.1.1 (NAD-abhängige ADH) und EC 1.1.1.2 (NADP-abhängige ADH) stammen.

Die zur Kofaktor-Regeneration eingesetzte NAD(P)H-Oxidase kann aus einer der Gruppen EC 1.6.3.1 (NAD(P)H-Oxidase (H₂O₂-bildend)), EC 1.6.3.2 (NAD(P)H-Oxidase (H₂O-bildend)), EC 1.6.3.3 (NADH-Oxidase (H₂O₂-bildend)) sowie EC 1.6.3.4 (NADH-Oxidase (H₂O-bildend)) stammen, wobei die H₂O-bildenden Klassen besonders bevorzugt sind.

Eine besonders bevorzugt eingesetzte H₂O-bildende NAD(P)H-Oxidase umfasst oder besteht vorzugsweise aus eine Aminosäuresequenz, die ausgewählt ist aus der Gruppe bestehend aus:
i) einer Aminosäuresequenz, die eine Identität zu SEQ ID Nr. 6 von mindestens 80% aufweist,
ii) einer Aminosäuresequenz, die durch eine Nukleinsäure kodiert wird, die eine Identität zu SEQ ID Nr. 5 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 5 bindet.

SEQ ID Nr. 5: 
ATGAGCAAAATTGTTATCGTGGGTGCAAATCATGCAGGCACCGCAGCAATTAATACCATTCTGGATAATTA
TGGCAGCGAAAATGAAGTGGTTGTGTTTGATCAGAATAGCAACATTAGCTTTCTGGGTTGTGGTATGGCAC
TGTGGATTGGTAAACAAATTAGCGGTCCGCAGGGTCTGTTTTATGCAGATAAAGAAAGCCTGGAAGCAAA
AGGTGCCAAAATCTATATGGAAAGTCCGGTTACCGCCATTGATTATGATGCAAAACGTGTTACCGCACTGG
TTAATGGTCAAGAACATGTTGAAAGCTACGAGAAACTGATTCTGGCAACCGGTAGCACCCCGATTCTGCCT
CCGATTAAAGGTGCAGCCATTAAAGAAGGTAGTCGCGATTTTGAAGCAACCCTGAAAAATCTGCAGTTCGT
GAAACTGTATCAGAATGCCGAAGATGTGATTAACAAACTGCAGGATAAAAGCCAGAATCTGAATCGTATT
GCAGTTGTTGGTGCAGGTTATATTGGTGTTGAACTGGCAGAAGCATTTAAACGTCTGGGTAAAGAAGTGA
TTCTGATTGACGTTGTTGATACCTGTCTGGCAGGTTATTATGATCAGGATCTGAGCGAAATGATGCGTCAG
 
AATCTGGAAGATCATGGTATCGAACTGGCATTTGGTGAAACCGTTAAAGCAATTGAAGGTGATGGTAAAG
TGGAACGTATTGTTACCGATAAAGCAAGCCATGATGTGGATATGGTTATTCTGGCAGTTGGTTTTCGTCCG
AATACAGCACTGGGTAATGCAAAACTGAAAACCTTTCGTAATGGTGCCTTTCTGGTGGATAAAAAACAAGA
AACCAGCATCCCGGATGTTTATGCAATTGGTGATTGTGCAACCGTGTATGATAATGCCATTAACGACACCA
ACTATATTGCACTGGCAAGCAATGCACTGCGTAGCGGTATTGTTGCAGGTCATAATGCAGCCGGTCATAAA
CTGGAAAGTCTGGGTGTTCAGGGTAGCAATGGTATTTCAATTTTTGGCCTGAATATGGTTAGCACCGGTCT
GACCCAAGAAAAAGCCAAACGTTTTGGTTATAATCCGGAAGTTACCGCCTTTACCGATTTTCAGAAAGCCA
GCTTTATCGAGCATGATAACTATCCGGTTACGCTGAAAATTGTGTATGACAAAGATAGCCGTCTGGTTCTG
GGTGCACAGATGGCCAGCAAAGAAGATATGAGCATGGGTATTCACATGTTTAGCCTGGCCATTCAAGAGA
AAGTTACCATTGAACGTCTGGCCCTGCTGGATTATTTCTTTCTGCCGCATTTTAATCAGCCGTACAACTATAT
GACCAAAGCAGCACTGAAAGCCAAATAA
SEQ ID Nr. 6:

MSKIVIVGANHAGTAAINTILDNYGSENEVVVFDQNSNISFLGCGMALWIGKQISGPQGLFYADKESLEAKGAKI
YMESPVTAIDYDAKRVTALVNGQEHVESYEKLILATGSTPILPPIKGAAIKEGSRDFEATLKNLQFVKLYQNAEDVI
NKLQDKSQNLNRIAVVGAGYIGVELAEAFKRLGKEVILIDVVDTCLAGYYDQDLSEMMRQNLEDHGIELAFGET
VKAIEGDGKVERIVTDKASHDVDMVILAVGFRPNTALGNAKLKTFRNGAFLVDKKQETSIPDVYAIGDCATVYD
NAINDTNYIALASNALRSGIVAGHNAAGHKLESLGVQGSNGISIFGLNMVSTGLTQEKAKRFGYNPEVTAFTDF
QKASFIEHDNYPVTLKIVYDKDSRLVLGAQMASKEDMSMGIHMFSLAIQEKVTIERLALLDYFFLPHFNQPYNY
MTKAALKAK

Die bevorzugt verwendete H₂O-bildende NAD(P)H-Oxidase umfasst oder besteht vorzugsweise aus einer Aminosäuresequenz, die eine Identität zu SEQ ID Nr. 6 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die H₂O-bildende NAD(P)H-Oxidase die Aminosäuresequenz SEQ ID Nr. 6 oder besteht aus dieser.

Alternativ umfasst die H₂O-bildende NAD(P)H-Oxidase vorzugsweise eine Aminosäuresequenz, die durch eine Nukleinsäure kodiert wird, die eine Identität zu SEQ ID Nr. 5 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die H₂O-bildende NAD(P)H-Oxidase kodiert, die Nukleinsäuresequenz SEQ ID Nr. 5 oder besteht aus dieser.

Ein weiterer Aspekt der vorliegenden Erfindung betrifft die Verwendung einer H₂O-bildende NAD(P)H-Oxidase zur Kofaktorregeneration (NAD(P)H zu NAD(P)⁺), welche eine Aminosäuresequenz umfasst oder daraus besteht, die ausgewählt ist aus der Gruppe bestehend aus:
i) einer Aminosäuresequenz, die eine Identität zu SEQ ID Nr. 6 von mindestens 80% aufweist,
ii) einer Aminosäuresequenz, die durch eine Nukleinsäure kodiert wird, die eine Identität zu SEQ ID Nr. 5 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 5 bindet.

### Materialien

D-Tagatose und D-Talitol wurden von TCI, D-Psicose wurde von TCI und Hunan Garden Naturals Inc. (China), D-Fructose, NADPH-Tetranatriumsalz, und Methanol wurden von PanReac AppliChem (ITW Reagents), Zinkchlorid, IPTG (Isopropyl-β-D-thiogalactopyranosid) wurden von Sigma-Aldrich, Magnesiumchlorid-Hexahydrat, Kaliumdihydrogenphosphat, di-Kaliumhydrogenphosphat, NAD⁺, NADH-Dinatriumsalz, NADP⁺-Dinatriumsalz sowie Natriumdodecylsulfat (SDS) wurden von Carl Roth und Triethanolamin (TEA) wurde von Chem-Lab NV bezogen.

### Produktion der Enzyme & Herstellung der Lysate

### Allgemeines zur Expression von rekombinanten Enzymen in E. coli

Für die rekombinante Enzymproduktion in einem *Escherichia coli*-Stamm wurde zunächst das zu exprimierende Gen in einer PCR unter der Verwendung der genomischen DNA oder deren synthetisch an die Codon-Verwendung von *E. coli* angepasstes Äquivalent als Matrize zusammen mit spezifischen Oligonukleotiden, die zusätzlich Erkennungssequenzen für Restriktionsendonukleasen tragen, amplifiziert und aus dem Reaktionsgemisch isoliert. Nach dem Nukleinsäure-Verdau mit den Restriktionsenzymen Sphl und Hindlll wurde das für das Target-Enzym kodierende Genfragment in das mit Sphl und Hindlll geschnittene Rückgrat des Expressionsvektors pQE70-Kan ligiert. Das Ligationsprodukt wurde in chemisch kompetente *E. coli*-Zellen Top10F transformiert und die entstandenen Kolonien wurden für die Plasmid-Isolierung und Restriktionsanalyse benutzt.

Das Ergebnis des Klonierungs-Schritts wurde mittels Restriktionsenzym-Verdau und DNA-Sequenzierung überprüft. Das resultierende Konstrukt trägt das Target-Gen unter dem IPTGinduzierbaren T5-Promotor.

Für die Überexpression des Enzymes in *E. coli* wurde das resultierende Expressionsplasmid in die kompetenten Expressionszellen RB791 transformiert. Nach 24 h Inkubation bei 37 °C wurden entstandene Kolonien für die Expressionstests in LB-Medium angeimpft.

Am nächsten Tag wurden damit Expressionskulturen mit einer optische Dichte OD₅₅₀ von 0,02 angeimpft und bei 37 °C geschüttelt, bis eine OD₅₅₀ von 0,3 erreicht wurde. Anschließend wurde die Temperatur auf 25 °C gesenkt und die Kulturen wurden beim Erreichen einer OD₅₅₀ von 0,5 mit 0,1 mM IPTG induziert. Nach 22 h wurden die Kulturen geerntet (vom Medium mittels Zentrifugation in Form eines Zellpellets abgetrennt) und auf die Expression des rekombinanten Enzymes mit Hilfe von SDS-Gelelektrophorese und einer Aktivitätsbestimmung (Verwendung in Use-Test oder optisch-enzymatischer Assay) analysiert.

### Herstellung von Zell-Lysaten mittels GEA-Aufschlusses

Zur Herstellung einer Zell-Suspension (Volumen > 50 ml) wurde das nach obigen Verfahren hergestellte Zellpellet in einem geeigneten Gefäß eingewogen und in Puffer (Triethanolamin (TEA) - HCl oder Kaliumphosphat-Puffer (KPP); siehe Tabelle 1 für verwendete Puffer-Systeme) gelöst. Der Massenanteil an Biomasse beträgt üblicherweise 20%, der Rest entfällt auf den Puffer.

Zum Zell-Aufschluss wurde ein Lab Homogenizer PandaPLUS 2000 von GEA verwendet (Druck ca. 1000 bar).

Die Homogenate wurden 10 min lang bei 4 °C und 16000 rpm zentrifugiert (Hermle Z 446 K Zentrifuge), um die unlöslichen Zellfragmente abzutrennen und das Lysat zu erhalten.

### Herstellung von Zell-Lysaten mittels Retsch-Aufschlusses

Zur Herstellung einer Zell-Suspension wurde das nach obigen Verfahren hergestellte Zellpellet (ca. 50 mg) in einem 2 ml Eppendorf-Vial eingewogen und mit Puffer (ca. 450 µl) versetzt (Tabelle 1 für verwendete Puffer-Systeme) und mittels Vortexens gelöst. Dadurch wird eine 10%ige Zell-Suspension erhalten.

Die Suspension wurde mit Glasperlen versetzt und in einer Retsch-Mühle MM 400 (24 Hz, 10 min) aufgeschlossen. Im Anschluss wurde das Homogenat für 1 min bei 4 °C und 16000 rpm zentrifugiert (Himac Zentrifuge CT 15RE), um das Lysat zu erhalten.

**Tabelle 1. Enzymklassen, Spenderorganismen und Aufschlussbedingungen für die in den Beispielen verwendeten Enzyme.**

| **Enzymtyp (EC-Klasse)** | **katalysierte Reaktion** | **Spenderorganismus** | **Aufschlussbedingungen** | **Literatur/SEQ ID Nr.** |
|---|---|---|---|---|
| D-Psicose-3-Epimerase (EC 5.1.3.30) | D-Fructose → D-Psicose | *Clostridium cellulolyticum* H10 | GEA (20% Biomasse in 100 mM TEA-HCl pH 7 + 2 mM MgCl₂) | (Mu et al., 2011; Chan et al., 2012) |
| Oxidoreduktase I (SEQ ID NO: 2) | D-Psicose <-> D-Talitol | | Retsch (10% Biomasse in 100 mM TEA-HCl pH 7); GEA (20% Biomasse in 100 mM TEA-HCl pH 7) | SEQ ID Nr. 2 |
| Oxidoreduktase II (SEQ ID NO: 4) | D-Talitol → D-Tagatose | | GEA (20% Biomasse in 100 mM TEA-HCl pH 7) | SEQ ID Nr. 4 |
| Alkoholdehydro genase (ADH) (EC 1.1.1.1) | 2-Propanol → Aceton | *(Geo-)Bacillus stearothermophilus* NCA1503 | GEA (20% Biomasse in 100 mM TEA-HCl pH 7 + 0,5 mM ZnCl₂) | (Sakoda & Imanaka, 1992) |
| NADH-Oxidase (EC 1.6.3.4) | NADH → NAD⁺ | *Streptococcus mutans* | GEA (20% Biomasse in 100 mM KPP pH 7) | (Matsumoto et al., 1996) |
| NAD(P)H-Oxidase (EC 1.6.3.2) | NAD(P)H → NAD(P)⁺ | *Streptococcus mutans;* Mutante | | (Petschacher et al., 2014) |

### Analytische Methoden

### High Performance Liquid Chromatography

Zur Quantifizierung von D-Psicose, D-Tagatose und D-Fructose sowie der Zwischenstufe D-Talitol mittels HPLC (High Performance Liquid Chromatography) wurde ein Agilent HPLC 1260 Infinity II Series System verwendet. Die Detektion erfolgte mittels eines Brechungsindex-Detektors (RI-Detektion). Zur Messung wurde eine Phenomenex Rezex RPM-Monosaccharide Pb+2 (8%) Säule mit entsprechender Vorsäule verwendet und mit Reinstwasser isokratisch eluiert.

### Bestimmung von Enzym-Aktivitäten (optisch-enzymatischer Assay)

Enzym-Aktivitäten in den Lysaten wurden mit einem Shimadzu UV-1900 Spektrophotometer bestimmt. Dazu wurde die Bildung bzw. der Verbrauch von NAD(P)H bei einer Wellenlänge von 340 nm über die Änderung der Absorption verfolgt. Die Messungen wurden mit 0,2 mM Kofaktor (NAD(P)⁺ oder NAD(P)H) durchgeführt. Dazu wurden 20 µl einer 10 mM Stock-Lösung des Kofaktors in einer Küvette (Greiner bio-one Semi-Micro-Küvette aus Polystyrol) vorgelegt und der gewünschte pH-Wert wurde mit 100 mM TEA-HCl-Puffer eingestellt (870 µl). 10 µl Lysat (verdünnt oder unverdünnt) und 100 µl Substrat-Lösung wurden zur Küvette zugesetzt und die Messung unmittelbar danach gestartet. Die Messungen wurden standardmäßig bei 25 °C durchgeführt. Über den Extinktionskoeffizienten von NADH bzw. NADPH bei 340 nm (*ε* = 6220 L mol⁻¹ cm⁻¹) kann die Enzym-Aktivität des Lysats in U/ml (bezogen auf das Volumen des Lysats) oder U/g (bezogen auf die zur Herstellung eingesetzte Biomasse) bestimmt werden. 1 U steht dabei für 1 µmol Substratumsatz pro Minute (1 U = 1 µmol/min = 1,67,10⁻⁸ kat).

Mit den nachfolgenden Beispielen werden bevorzugte Varianten des erfindungsgemäßen Verfahrens noch näher beschrieben. Die in diesen Beispielen eingesetzten Lysate wurden nach den oben beschriebenen Verfahren hergestellt.

### Beispiel 1

### Herstellung von D-Talitol aus D-Fructose

Die Reaktion wurde in einem Labfors 5 Tisch-Bioreaktor (Infors AG) durchgeführt. Als Gefäß wurde ein Glasreaktor (Volumen 3,4 l) mit aufgesetztem Rührwerk, pH-Elektrode und O₂-Sensor verwendet. Die pH-Kontrolle erfolgte durch die Zudosierung von 1M NaOH oder 1M H₂SO₄.

Zu Beginn wurden 50 ml einer D-Fructose-Lösung (500 g/l), 238,7 ml deionisiertes Wasser, 89 ml eines 200 mM TEA-HCl-Puffers (pH 7,5) im Reaktor vorgelegt und unter Rühren auf 35 °C gebracht.

Zum Start der Reaktion wurden 25 ml D-Psicose-3-Epimerase-Lysat zugesetzt. Danach wurden 35 ml Oxidoreduktase I-Lysat (GEA-Lysat), 8 kU Alkoholdehydrogenase-Lysat, 10 ml einer 10 mM NAD⁺-Lösung sowie 40 ml 2-Propanol eingebracht.

Während des Betriebs wurden laufend Proben von der Reaktorlösung genommen, die folgendermaßen analysiert wurden: 100 µl der Reaktorlösung wurden mit 200 µl Methanol versetzt und im Eppendorf Thermomixer bei 60 °C und 1200 rpm für 15 min inkubiert. Die Probe wurde in einer Zentrifuge kurz zentrifugiert, mit 700 µl deionisiertem Wasser versetzt, gevortext und anschließend für 5 min bei max. g zentrifugiert. 200 µl des Überstands wurden in ein HPLC-Vial mit Insert überführt und mittels HPLC (Rl-Detektion) vermessen.

Nach 24 h und 42 h Laufzeit wurden je 15 ml 2-Propanol nachdosiert.

Nach 67 h wurden 98,0% der D-Fructose zu D-Talitol umgesetzt (gefundene Konzentration: 45,1 g/l).

Im Anschluss wurde der Reaktorinhalt unter Rührung für 30 min auf 70 °C erhitzt. Die heiße Mischung wurde durch einen Faltenfilter (Schleicher & Schuell 595 ½, 185 mm) filtriert. Das Filtrat wurde mit Aktivkohle für 30 min bei 50 °C gerührt, wonach die Aktivkohle abfiltriert wurde (Faltenfilter Schleicher & Schuell 595 ½, 185 mm). Die farblose Lösung wurde mit einem Mischbett-Ionentauscherharz (Amberlite MB20) für 30 min bei 50 °C behandelt, welches danach durch Filtration (Faltenfilter Schleicher & Schuell 595 ½, 185 mm) entfernt wurde. Am Rotationsverdampfer (60 °C, 150 - 60 mbar) wurde die Lösung bis zu einer Konzentration von ca. 600 g/l D-Talitol aufkonzentriert. Der entstandene Sirup wurde über Nacht bei 4 °C im Kühlschrank inkubiert. Da keine Kristallbildung beobachtet werden konnte, wurde ein Impfkristall zugesetzt und der Sirup wieder über Nacht bei 4 °C im Kühlschrank gelagert. Der Sirup wurde mit demselben Volumen an IPA versetzt und bei -20 °C im Tiefkühlschrank gelagert, um die Kristallisation auszulösen. Die farblosen Kristalle wurden nach Anlegen eines Vakuums durch eine Glasfritte (P4, 10-16 µm) abfiltriert und 24 h bei 50 °C im Vakuumtrockenschrank getrocknet. Eine HPLC-Analyse der Kristalle (50 % Ausbeute) ergab, dass es sich bei dem Produkt um D-Talitol (Reinheit 94%) handelte.

### Beispiel 2

### Oxidation von D-Talitol zu D-Tagatose

In einem 2 ml Glas-Vial wurden folgende Komponenten vermischt: 147,2 µl deionisiertes Wasser, 125 µl eines 400 mM TEA-HCl-Puffers (pH 8,5), 150 µl einer D-Talitol-Lösung (168 g/l), 35 µl Oxidoreduktase II-Lysat, 10 U NAD(P)H-Oxidase-Lysat sowie 5 µl einer 5 mM NADP⁺-Lösung. Der Ansatz wurde in einem Eppendorf-Thermomixer unter kontinuierlichem Schütteln (24°C, 800 rpm) für 20 h inkubiert.

Zur Analyse wurden 100 µl des Ansatzes mit 200 µl Methanol versetzt und im Eppendorf-Thermomixer bei 60 °C und 1200 rpm für 15 min inkubiert. Die Probe wurde in einer Zentrifuge kurz zentrifugiert, mit 700 µl deionisiertem Wasser versetzt, gevortext und anschließend für 5 min bei max. g zentrifugiert. 200 µl des Überstands wurden in ein HPLC-Vial mit Insert überführt und mittels HPLC (RI-Detektion) vermessen.

Auf diese Weise wurden 81,7% des D-Talitols zu D-Tagatose (gefundene Konzentration: 47,7 g/l) umgesetzt.

### Beispiel 3

### Herstellung von D-Tagatose aus D-Fructose (Eintopf-Verfahren)

Die Reaktion wurde in einem Labfors 5 Tisch-Bioreaktor (Infors AG) durchgeführt. Als Gefäß wurde ein Glasreaktor (Volumen 3,4 l) mit aufgesetztem Rührwerk, pH-Elektrode und O₂-Sensor verwendet. Die pH-Kontrolle erfolgte durch die Zudosierung von 1M NaOH oder 1M H₂SO₄.

Zu Beginn wurden 50 ml einer D-Fructose-Lösung (500 g/l), 238,8 ml deionisiertes Wasser, 89 ml eines 200 mM TEA-HCl-Puffers (pH 7,5) im Reaktor vorgelegt und unter Rühren auf 35 °C gebracht.

Zum Start der Reaktion wurden 25 ml D-Psicose-3-Epimerase-Lysat zugesetzt. Danach wurden 35 ml Oxidoreduktase I-Lysat (GEA-Lysat), 8 kU Alkoholdehydrogenase-Lysat, 10 ml einer 10 mM NAD⁺-Lösung sowie 40 ml 2-Propanol eingebracht.

Während des Betriebs wurden laufend Proben von der Reaktorlösung genommen, die folgendermaßen analysiert wurden: 100 µl der Reaktorlösung wurden mit 200 µl Methanol versetzt und im Eppendorf Thermomixer bei 60 °C und 1200 rpm für 15 min inkubiert. Die Probe wurde in einer Zentrifuge kurz zentrifugiert, mit 700 µl deionisiertem Wasser versetzt, gevortext und anschließend für 5 min bei max. g zentrifugiert. 200 µl des Überstands wurden in ein HPLC-Vial mit Insert überführt und mittels HPLC (Rl-Detektion) vermessen.

Nach 24 h Laufzeit wurden 15 ml 2-Propanol nachdosiert.

Nach 47 h wurden 94,0% der D-Fructose zu D-Talitol umgesetzt (gefundene Konzentration: 41,3 g/l).

Nach Abkühlen des Reaktorinhalts auf 24 °C wurden 15 ml Oxidoreduktase II-Lysat, 5 kU NAD(P)H-Oxidase-Lysat sowie 10 ml einer 5 mM NADP⁺-Lösung zugesetzt. Zusätzlich wurde der pH-Wert der Reaktorlösung auf 8 erhöht.

Nach 97 h Gesamtlaufzeit wurde der Reaktorinhalt auf 70 °C erhitzt, der pH-Wert auf 4 eingestellt und für 30 min bei 70 °C gerührt. Der Niederschlag wurde über eine Glasfritte (P3) abfiltriert.

Im Filtrat wurden 29,9 g/l D-Tagatose detektiert.

Das Filtrat wurde bis zu einer D-Tagatose-Konzentration von 257 g/l am Rotationsverdampfer aufkonzentriert.

### Beispiel 4

### Oxidation von D-Talitol zu D-Psicose

In einem 2 ml Glas-Vial wurden folgende Komponenten vermischt: 30 µl deionisiertes Wasser, 250 µl eines 200 mM TEA-HCl-Puffers (pH 8), 125 µl einer D-Talitol-Lösung (200 g/l), 50 µl Oxidoreduktase I-Lysat (Retsch-Lysat), 40 µl NADH-Oxidase-Lysat sowie 5 µl einer 10 mM NAD⁺-Lösung. Der Ansatz wurde in einem Eppendorf-Thermomixer unter kontinuierlichem Schütteln (30 °C, 800 rpm) für 20 h inkubiert.

Zur Analyse wurden 100 µl des Ansatzes mit 200 µl Methanol versetzt und im Eppendorf-Thermomixer bei 60 °C und 1200 rpm für 15 min inkubiert. Die Probe wurde in einer Zentrifuge kurz zentrifugiert, mit 700 µl deionisiertem Wasser versetzt, gevortext und anschließend für 5 min bei max. g zentrifugiert. 200 µl des Überstands wurden in ein HPLC-Vial mit Insert überführt und mittels HPLC (RI-Detektion) vermessen.

Auf diese Weise wurden 75,3% des D-Talitols zu D-Psicose (gefundene Konzentration: 39,3 g/l) umgesetzt.

### Beispiel 5

### Herstellung von D-Psicose aus D-Fructose (Eintopf-Verfahren)

In einem 2 ml Glas-Vial wurden folgende Komponenten vermischt: 73,8 µl deionisiertes Wasser, 250 µl eines 200 mM TEA-HCl-Puffers (pH 7,5), 50 µl einer D-Fructose-Lösung (500 g/l) sowie 25 µl D-Psicose-3-Epimerase-Lysat. Danach wurden 35 µl Oxidoreduktase I-Lysat (GEA-Lysat), 8 U Alkoholdehydrogenase-Lysat, 5 µl einer 10 mM NAD⁺-Lösung sowie 50 µl 2-Propanol zum Ansatz hinzugefügt. Der Ansatz wurde unter kontinuierlichem Schütteln (35 °C, 800 rpm) für insgesamt 20 h inkubiert.

Danach wurde der Ansatz 60 min lang auf 70 °C erhitzt. Nach Abkühlen wurden 25 µl Oxidoreduktase I-Lysat (GEA-Lysat), 10 U NADH-Oxidase-Lysat, 5 µl einer 5 mM NAD⁺-Lösung sowie 100 ml deionisiertes Wasser zugesetzt. Der Ansatz wurde in einem Eppendorf-Thermomixer unter kontinuierlichem Schütteln (24 °C, 800 rpm) für weitere 20 h inkubiert.

Zur Analyse wurden 100 µl des Ansatzes mit 200 µl Methanol versetzt und im Eppendorf-Thermomixer bei 60 °C und 1200 rpm für 15 min inkubiert. Die Probe wurde in einer Zentrifuge kurz zentrifugiert, mit 700 µl deionisiertem Wasser versetzt, gevortext und anschließend für 5 min bei max. g zentrifugiert. 200 µl des Überstands wurden in ein HPLC-Vial mit Insert überführt und mittels HPLC (RI-Detektion) vermessen.

Auf diese Weise wurden 76,9% der D-Fructose (50 g/l) zu D-Psicose umgesetzt (gefundene Konzentration: 39,9 g/l).

### Literatur

Reed, R. H., Wright, P. J., Chudek, J. A., & Hunter, G. (1995). Turnover of hexitols in the marine macroalga Himanthalia elongata (Phaeophyta, Fucales). European Journal of Phycology, 30(3), 169-177. https://doi.org/10.1080/09670269500650951
Izumori, K. (2006). Izumoring: a strategy for bioproduction of all hexoses. Journal of Biotechnology, 124(4), 717-722. https://doi.org/10.1016/j.jbiotec.2006.04.016
Hirst, E. L., Hough, L., & Jones, J. K. N. (1949). Composition of the Gum of Sterculia setigera: Occurrence of D-Tagatose in Nature. Nature, 163, 177. https://doi.org/10.1038/163177b0
Lindberg, B. (1955). Studies on the Chemistry of Lichens. VIII. Investigation of a Dermatocarpon and Some Roccella Species. Acta Chemica Scandinavica, 9, 917-919. https://doi.org/10.3891/acta.chem.scand.09-0917
Adachi, S. (1958). Formation of Lactulose and Tagatose from Lactose in strongly heated Milk. Nature, 181, 840-841 (1958). https://doi.org/10.1038/181840a0
Hough, L., & Stacey, B. E. (1963). The occurrence of D-ribohexulose in Itea ilicifolia, Itea virginica, and Itea yunnanensis. Phytochemistry, 2(4), 315-320. https://doi.org/10.1016/S0031-9422(00)84854-2
Oshima, H., Kimura, I., & Izumori, K. (2006). Psicose Contents in Various Food Products and its Origin. Food Science and Technology Research, 12(2), 137-143. https://doi.org/10.3136/fstr.12.137
Jiang, S., Xiao, W., Zhu, X., Yang, P., Zheng, Z., Lu, S., Jiang, S., Zhang, G., & Liu, J. (2020). Review on D-Allulose: In vivo Metabolism, Catalytic Mechanism, Engineering Strain Construction, Bio-Production Technology. Frontiers in Bioengineering and Biotechnology, 8, 26. https://doi.org/10.3389/fbioe.2020.00026
Oh, D.-K. (2007). Tagatose: properties, applications, and biotechnological processes. Applied Microbiology and Biotechnology, 76, 1-8. https://doi.org/10.1007/s00253-007-0981-1
Roy, S., Chikkerur, J., Roy, S. C., Dhali, A., Kolte, A. P., Sridhar, M., & Samanta, A. K. (2018). Tagatose as a Potential Nutraceutical: Production, Properties, Biological Roles, and Applications. Journal of Food Science, 83(11), 2699-2709. https://doi.org/10.1111/1750-3841.14358
Levin, G. V. (2002). Tagatose, the New GRAS Sweetener and Health Product. Journal of Medical Food, 5(1), 23-36. https://doi.org/10.1089/109662002753723197
Ahmed, A., Khan, T. A., Ramdath, D. D., Kendall, C. W. C., & Sievenpiper, J. L. (2022). Rare sugars and their health effects in humans: a systematic review and narrative synthesis of the evidence from human trials, Nutrition Reviews, 80(2), 255-270. https://doi.org/10.1093/nutrit/nuab012
Mochizuki, S., Fukumoto, T., Ohara, T., Ohtani, K., Yoshihara, A., Shigematsu, Y., Tanaka, K., Ebihara, K., Tajima, S., Gomi, K., Ichimura, K., Izumori. K., & Akimitsu, K. (2020). The rare sugar D-tagatose protects plants from downy mildews and is a safe fungicidal agrochemical. Communications Biology, 3, 423. https://doi.org/10.1038/s42003-020-01133-7
Chahed, A., Nesler, A., Aziz, A., Barka, E. A., Pertot, I., & Perazzolli, M. (2021). A review of knowledge on the mechanisms of action of the rare sugar D-tagatose against phytopathogenic oomycetes. Plant Pathology, 70(9), 1979-1986. https://doi.org/10.1111/ppa.13440
Zhang, W., Yu, S., Zhang, T., Jiang, B., & Mu, W. (2016). Recent advances in D-allulose: Physiological functionalities, applications, and biological production. Trends in Food Science and Technology, 54, 127-137. https://doi.org/10.1016/j.tifs.2016.06.004
Chen, Z., Gao, X.-D., & Li, Z. (2022). Recent Advances Regarding the Physiological Functions and Biosynthesis of D-Allulose. Frontiers in Microbiology, 13, 881037. https://doi.org/10.3389/fmicb.2022.881037
Heath, E. C., Horecker, B. L., Smyrniotis, P. Z., & Takagi, Y. (1958). Pentose fermentation by Lactobacillus plantarum. II. L-arabinose isomerase. Journal of Biological Chemistry, 231(2), 1031-1037 (1958). https://doi.org/10.1016/S0021-9258(18)70464-X
Dai, Y., Zhang, J., Zhang, T., Chen, J., Hassanin, H. A. M., & Jiang, B. (2020). Characteristics of a fructose 6-phosphate 4-epimerase from Caldilinea aerophila DSM 14535 and its application for biosynthesis of tagatose. Enzyme and Microbial Technology, 139, 109594. https://doi.org/10.1016/j.enzmictec.2020.109594
Lee, S.-H., Hong, S.-H., Kim, K.-R., & Oh, D.-K. (2017). High-yield production of pure tagatose from fructose by a three-step enzymatic cascade reaction. Biotechnology Letters, 39, 1141-1148. https://doi.org/10.1007/s10529-017-2340-3
Shaw, D. R. D. (1956). Polyol dehydrogenases. 3. Galactitol dehydrogenase and D-iditol dehydrogenase. Biochemical Journal, 64(3), 394-405. https://doi.org/10.1042/bj0640394
Izumori, K., Khan, A. R., Okaya, H., & Tsumura, T. (1993). A New Enzyme, D-Ketohexose 3-Epimerase, from Pseudomonas sp. ST-24. Bioscience, Biotechnology, and Biochemistry, 57(6), 1037-1039. https://doi.org/10.1271/bbb.57.1037
Van Duc Long, N., Le, T.-H., Kim, J.-I., Lee, J. W. and Koo, Y.-M. (2009). Separation of D-psicose and D-fructose using simulated moving bed chromatography. Journal of Separation Science, 32(11), 1987-1995. https://doi.org/10.1002/jssc.200800753
Li, Y., Shi, T., Han, P., & You, C. (2021). Thermodynamics-Driven Production of Value-Added D-Allulose from Inexpensive Starch by an In Vitro EnzymaticSynthetic Biosystem. ACS Catalysis, 11(9), 5088-5099. https://doi.org/10.1021/acscatal.0c05718
Wang, W., Yang, J., Sun, Y., Li, Z., & You, C. (2020). Artificial ATP-Free in Vitro Synthetic Enzymatic Biosystems Facilitate Aldolase-Mediated C-C Bond Formation for Biomanufacturing. ACS Catalysis, 10(2), 1264-1271. https://doi.org/10.1021/acscatal.9b04696
Li, Z., Li, F., Cai, L., Chen, Z., Qin, L., & Gao, X.-D. (2020). One-Pot Multienzyme Synthesis of Rare Ketoses from Glycerol. Journal of Agricultural and Food Chemistry, 68(5), 1347-1353. https://doi.org/10.1021/acs.jafc.9b06748
Xiao, Q., Niu, J., Liu, H., Liu, Y., & Zhou, X. (2019). High Conversion of D-Fructose into D-Allulose by Enzymes Coupling with an ATP Regeneration System. Molecular Biotechnology, 61, 432-441. https://doi.org/10.1007/s12033-019-00174-6
Takeshita, K., Ishida, Y., Takada, G., & Izumori, K. (2000). Direct Production of Allitol from D-Fructose by a Coupling Reaction Using D-Tagatose 3-Epimerase, Ribitol Dehydrogenase and Formate Dehydrogenase. Journal of Bioscience and Bioengineering, 90(5), 545-548. https://doi.org/10.1016/51389-1723(01)80038-4
Sasahara, H., Mine, M., & Izumori, K. (1998). Production of D-talitol from D-psicose by Candida famata R28. Journal of Fermentation and Bioengineering, 85(1), 84-88. https://doi.org/10.1016/S0922-338X(97)80359-5
Muniruzzaman, S., Kobayashi, H., & Izumori, K. (1994). Production of D-talitol from D-tagatose by Aureobasidium pullulans strain 113B. Journal of Fermentation and Bioengineering, 78(5), 346-350. https://doi.org/10.1016/0922-338X(94)90278-X
Izumori, K., Yamakita, M., Tsumura, T., & Kobayashi, H. (1990). Production of D-psicose from D-talitol, D-tagatose or D-galactitol by Alcaligenes sp. 701B. Journal of Fermentation and Bioengineering, 70(1), 26-29. https://doi.org/10.1016/0922-338X(90)90025-R
Yoshihara, K., Shinohara, Y., Hirotsu, T., & Izumori, K. (2006). Bioconversion of D-psicose to D-tagatose and D-talitol by Mucoraceae fungi. Journal of Bioscience & Bioengineering, 101(3), 219-222. https://doi.org/10.1263/jbb.101.219
Wichelecki, D. J., Vetting, M. W., Chou, L., Al-Obaidi, N., Bouvier, J. T., Almo, S. C., & Gerlt, J. A. (2015). ATP-binding Cassette (ABC) Transport System Solute-binding Protein-guided Identification of Novel D-Altritol and Galactitol Catabolic Pathways in Agrobacterium tumefaciens C58. The Journal of Biological Chemistry, 290(48), 28963-28976. https://doi.org/10.1074/jbc.M115.686857
Shin, K.-C., Lee, T.-E., Seo, M.-J., Kim, D. W., Kang, L.-W. & Oh, D.-K. (2020). Development of Tagaturonate 3-Epimerase into Tagatose 4-Epimerase with a Biocatalytic Route from Fructose to Tagatose. ACS Catalysis, 10(20), 12212-12222. https://doi.org/10.1021/acscatal.0c02922
Jeon, E. J., Lee, Y.-M., Choi, E. J., Kim, S.-B., & Jeong, K. J. (2023). Production of Tagatose by Whole-cell Bioconversion from Fructose Using Corynebacterium glutamicum. Biotechnology and Bioprocess Engineering. https://doi.org/10.1007/s12257-022-0304-5
Wang, L., Chen, K., Zheng, P., Huo, X., Liao, F., Zhu, L., Hu, M., & Tao, Y. (2023). Enhanced production of D-psicose from D-fructose by a redox-driven multi-enzyme cascade system. Enzyme and Microbial Technology, 163, 110172. https://doi.org/10.1016/j.enzmictec.2022.110172
Lin, B., & Tao, Y. (2017). Whole-cell biocatalysts by design. Microbial Cell Factories, 16, 106. https://doi.org/10.1186/s12934-017-0724-7
Altschul, S. F., Gish, W., Miller, W., Myers, E. W., & Lipman, D. J. (1990). Basic local alignment search tool. Journal of Molecular Biology, 215(3), 403-410. https://doi.org/10.1016/S0022-2836(05)80360-2
Henikoff, S., & Henikoff, J. G. (1992). Amino acid substitution matrices from protein blocks. Proceedings of the National Academy of Sciences of the United States of America, 89(22), 10915-10919. https://doi.org/10.1073/pnas.89.22.10915
Sambrook, J., Fritsch, E. R., & Maniatis, T. (1989). Molecular Cloning: A Laboratory Manual (2nd ed.). Cold Spring Harbor, NY: Cold Spring Harbor Laboratory Press.
Mu, W., Chu, F., Xing, Q., Yu, S., Zhou, L., & Jiang, B. (2011). Cloning, Expression, and Characterization of a D-Psicose 3-Epimerase from Clostridium cellulolyticum H10. Journal of Agricultural and Food Chemistry, 59(14), 7785-7792. https://doi.org/10.1021/jf201356q
Chan, H.-C., Zhu, Y., Hu, Y., Ko, T.-P., Huang, C.-H., Ren, F., Chen, C.-C., Ma, Y., Guo, R.-T., & Sun, Y. (2012). Crystal structures of D-psicose 3-epimerase from Clostridium cellulolyticum H10 and its complex with ketohexose sugars. Protein & Cell, 3(2), 123-131. https://doi.org/10.1007/s13238-012-2026-5
Sakoda, H., & Imanaka, T. (1992). Cloning and sequencing of the gene coding for alcohol dehydrogenase of Bacillus stearothermophilus and rational shift of the optimum pH. Journal of Bacteriology, 174(4), 1397-1402. https://doi.org/10.1128/jb.174.4.1397-1402.1992
Matsumoto, J., Higuchi, M., Shimada, M., Yamamoto, Y., & Kamio, Y. (1996). Molecular Cloning and Sequence Analysis of the Gene Encoding the H2O-forming NADH Oxidase from Streptococcus mutans. Bioscience, Biotechnology, and Biochemistry, 60(1), 39-43. https://doi.org/10.1271/bbb.60.39
Petschacher, B., Staunig, N., Müller, M., Schürrmann, M., Mink, D., De Wildeman, S., Gruber, K., & Glieder, A. (2014). COFACTOR SPECIFICITY ENGINEERING OF STREPTOCOCCUS MUTANS NADH OXIDASE 2 FOR NAD(P)+ REGENERATION IN BIOCATALYTIC OXIDATIONS. Computational and Structural Biotechnology Journal, 9(14), e201402005. https://doi.org/10.5936/csbj.201402005

## Patentansprüche

1. Verfahren zur Herstellung einer wässerigen Lösung enthaltend D-Talitol, indem aus D-Fructose, welche in einer wässerigen Lösung gelöst vorliegt, durch Behandeln mit einer Epimerase *in vitro* D-Psicose gebildet wird, wonach die D-Psicose durch Behandeln mit einer NAD(P)H-abhängigen Oxidoreduktase *in vitro* zu D-Talitol reduziert und die Epimerase und die Oxidoreduktase abgetrennt werden.

2. Verfahren nach Anspruch 1, **dadurch gekennzeichnet, dass** der durch die Reduktion entstandene oxidierte Kofaktor NAD(P)⁺ mittels einer Alkoholdehydrogenase und einem sekundären Alkohol unter Bildung eines Ketons reduziert wird.

3. Verfahren nach Anspruch 2, **dadurch gekennzeichnet, dass** der sekundäre Alkohol 2-Propanol ist.

4. Verfahren nach einem der Ansprüche 1 bis 3, **dadurch gekennzeichnet, dass** die NAD(P)H-abhängige Oxidoreduktase zur Reduktion von D-Psicose zu D-Talitol eine Aminosäuresequenz umfasst, welche ausgewählt ist aus der Gruppe bestehend aus:
i) einer Aminosäuresequenz, die eine Identität zu SEQ ID Nr. 2 von mindestens 80% aufweist,
ii) einer Aminosäuresequenz, die durch eine Nukleinsäure kodiert wird, die eine Identität zu SEQ ID Nr. 1 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet.

5. Verfahren zur Herstellung von D-Talitol oder D-Psicose umfassend den Schritt des Behandelns von D-Psicose oder D-Talitol mit einer Oxidoreduktase, die eine Aminosäuresequenz umfasst, welche ausgewählt ist aus der Gruppe bestehend aus:
i) einer Aminosäuresequenz, die eine Identität zu SEQ ID Nr. 2 von mindestens 80% aufweist,
ii) einer Aminosäuresequenz, die durch eine Nukleinsäure kodiert wird, die eine Identität zu SEQ ID Nr. 1 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet.

6. Oxidoreduktase zur Reduktion von D-Psicose zu D-Talitol oder zur Oxidation von D-Talitol zu D-Psicose umfassend eine Aminosäuresequenz, welche ausgewählt ist aus der Gruppe bestehend aus:
i) einer Aminosäuresequenz, die eine Identität zu SEQ ID Nr. 2 von mindestens 80% aufweist,
ii) einer Aminosäuresequenz, die durch eine Nukleinsäure kodiert wird, die eine Identität zu SEQ ID Nr. 1 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet.

7. Verfahren zur Herstellung einer wässerigen Lösung von D-Tagatose oder D-Psicose, **dadurch gekennzeichnet, dass** die nach den Verfahren nach den Patentansprüchen 1 bis 4 hergestellte, wässerige, D-Talitol enthaltende Lösung mit einer NAD(P)⁺-abhängigen Oxidoreduktase behandelt wird, um D-Talitol zu D-Tagatose bzw. D-Talitol zu D-Psicose zu oxidieren, wonach die Oxidoreduktase abgetrennt wird.

8. Verfahren nach Anspruch 7, **dadurch gekennzeichnet, dass** der durch die Oxidation entstandene, reduzierte Kofaktor NAD(P)H mittels einer Oxidase und Sauerstoff zu NAD(P)⁺ oxidiert wird.

9. Verfahren nach Anspruch 8, **dadurch gekennzeichnet, dass** die Oxidase zur Oxidation von Kofaktor NAD(P)H zu NAD(P)⁺ eine Aminosäuresequenz umfasst, die ausgewählt ist aus der Gruppe bestehend aus:
i) einer Aminosäuresequenz, die eine Identität zu SEQ ID Nr. 6 von mindestens 80% aufweist,
ii) einer Aminosäuresequenz, die durch eine Nukleinsäure kodiert wird, die eine Identität zu SEQ ID Nr. 5 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 5 bindet.

10. Verfahren nach einem der Ansprüche 1 bis 8, **dadurch gekennzeichnet, dass** es als Eintopf-Reaktion ohne Isolierung von Zwischenprodukten durchgeführt wird.

11. Verfahren nach einem der Ansprüche 1 bis 9, **dadurch gekennzeichnet, dass** die Enzyme als Homogenat oder Lysat oder Lyophilisat der entsprechenden, sie bildenden Zellen vorliegen.

12. Verfahren nach einem der Ansprüche 7 bis 10, **dadurch gekennzeichnet, dass** die NAD(P)⁺-abhängige Oxidoreduktase zur Oxidation von D-Talitol zu D-Tagatose eine Aminosäuresequenz umfasst, welche ausgewählt ist aus der Gruppe bestehend aus:
i) einer Aminosäuresequenz, die eine Identität zu SEQ ID Nr. 4 von mindestens 80% aufweist,
ii) einer Aminosäuresequenz, die durch eine Nukleinsäure kodiert wird, die eine Identität zu SEQ ID Nr. 3 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 3 bindet.

13. Verfahren nach einem der Ansprüche 7 bis 10, **dadurch gekennzeichnet, dass** die NAD(P)⁺-abhängige Oxidoreduktase zur Oxidation von D-Talitol zu D-Psicose eine Aminosäuresequenz umfasst, welche ausgewählt ist aus der Gruppe bestehend aus:
i) einer Aminosäuresequenz, die eine Identität zu SEQ ID Nr. 2 von mindestens 80% aufweist,
ii) einer Aminosäuresequenz, die durch eine Nukleinsäure kodiert wird, die eine Identität zu SEQ ID Nr. 1 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet.

14. Verfahren zur Herstellung von D-Tagatose umfassend den Schritt des Behandelns von D-Talitol mit einer Oxidoreduktase, die eine Aminosäuresequenz umfasst, welche ausgewählt ist aus der Gruppe bestehend aus:
i) einer Aminosäuresequenz, die eine Identität zu SEQ ID Nr. 4 von mindestens 80% aufweist,
ii) einer Aminosäuresequenz, die durch eine Nukleinsäure kodiert wird, die eine Identität zu SEQ ID Nr. 3 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 3 bindet.

15. Oxidoreduktase zur Oxidation von D-Talitol zu D-Tagatose umfassend eine Aminosäuresequenz, welche ausgewählt ist aus der Gruppe bestehend aus:
i) einer Aminosäuresequenz, die eine Identität zu SEQ ID Nr. 4 von mindestens 80% aufweist,
ii) einer Aminosäuresequenz, die durch eine Nukleinsäure kodiert wird, die eine Identität zu SEQ ID Nr. 3 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 3 bindet.
